# EUROPEAN PATENT APPLICATION

(11) **EP 4 450 604 A2**
(43) Date of publication of application: **23.10.2024**
(21) Application number: 24191695.6
(22) Date of filing: 22.04.2022
(51) Int. Cl.: C11B 9/00

(54) **TRANSPARENT DIFFUSIBLE MICROEMULSIONS**

(62) Divisional of application: 22725398.6
(71) Applicant: Symrise AG, 37603 Holzminden Niedersachsen (DE)
(72) Inventor: KAUFHOLD, Astrid, 37603 Holzminden (DE); VON HOLLY-PONIENTZIETZ, Julia, 37603 Holzminden (DE); HARZKE, Falk, 37619 Rühle (DE); WINGERTER, Christine, 37603 Holzminden (DE)
(74) Representative: Fabry, Bernd

(57) **Abstract**

Suggested is a transparent diffusible microemulsion comprising a non-aqueous and an aqueous phase, said non-aqueous phase comprising or consisting of
(a) at least one fragrance or perfume oil;
(b) at least one nonionic solubilizer;
(c) at least one solvent selected from the group consisting of 1,2-propylenglycol; 3-methoxy-3-methyl-1-butanol (MMB), dipropyleneglycol (DPG), dipropyleneglycol methyl ether (DPM), dipropylene glycol methyl ether acetate (DPMA), and tripropylenglycol methyl ether (TPM); and
(d) 1,2-pentanediol.

## Description

### AREA OF INVENTION

The present invention refers to the area of fragrances and perfumes and relates to a transparent diffusible microemulsion useful for reed diffusors.

### BACKGROUND OF THE INVENTION

Reed diffusors (also called aroma diffusers) have experienced a strong demand in recent years, as they not only improve the air in the room, but with exotic scents such as lemongrass, bamboo or vanilla are also able to evoke special pleasant moods, for example of vacation or Christmas time. A distinction is made between diffusers in which the evaporation of the perfume oil takes place by supplying heat (either directly or electrically) and diffusion through reeds plastic fiber sticks or paper.

Reed diffuser compositions are typically based on expensive and sometimes ecologically harmful solvents such as for example dipropyleneglycol monomethyl ether (DPM). For this reason, perfume industry is looking for alternatives, preferably water-based systems. Water-based systems, however, mostly do not work technically or allow only low perfume contents. This causes a poor room impact in use. To get a similar fragrance performance for common systems, the same amount of perfume must be given to the water phase. To get this perfume content soluble it is necessary to increase the solubilizer content significantly. Unfortunately, with huge amounts of non-evaporating solubilizers the wick will be cogged after very short time in use and no further evaporation is possible.

### RELEVANT PRIOR ART

EP 2629807 B1 (AGILEX) claims a liquid reed diffusor fragrance composition comprising: (a) 0.1% to 60% by weight of an oil phase comprising a fragrance; (b) at least 10% by weight of water; (c) a fragrance vehicle comprising at least one mono-, di-, or polyglycol ether; and (d) an amphiphilic solubilizing agent.

EP 2810663 B1 (RECKITT) describes a fragrance composition comprising at least two different fragrance accords wherein at least 30 wt.-percent of each fragrance accord comprises the key contributor(s) of said fragrance accord and wherein the average of the odor detection thresholds of said key contributor(s) for each fragrance accord is within the same order of magnitude as the average of the odor detection thresholds of the key contributor(s) for said other fragrance accord(s); and wherein the base note(s) of each fragrance accord comprises less than 15 percent of the notes of said accord. The composition is particular useful for reed diffusors.

WO 2016 1196601 A1 (RHODIA) refers to a fragrance composition comprising a fragrance compound and a solvent or carrier, wherein at least 10 percent by weight based on the total weight of solvent and carrier of the fragrance composition is one or more specific low vapor pressure volatile organic compound (VOC). The new fragrance composition is particularly well adapted to air care devices like for example reed diffusors.

GB 2474042 B2 (BARKLETT) claims a method of blending valerian oil with other essential oils plus water and a dispersing agent, using a reed diffuser to provide slow release of the composition properties into the environment for the purpose of helping to relieve stress and its associated symptoms in humans and other mammals. Preferably the composition contains valerian, vetivert, sweet basil and sage oils. The preferred dispersing agent is 3-methyl-3-methoxybutyl acetate.

### OBJECT OF THE INVENTION

Therefore, it has been the object of the present invention providing new water-based formulations, which enhance the amount of addable perfume-oil with low solubilizer content and a combination of solvents in small amounts to optimize the solubility of the perfume. In addition, the composition should be stable even when stored at 40 °C and 4°C for a period of 12 weeks. Finally, the composition should increase the recognition of the heart notes of the perfume compositions. More concrete, it has been the object of the invention developing a perfect blend of solubilizer and solvent for reducing the amount of solubilizer, particularly to avoid blocking of the sticks, while the olfactory sensation is maintained.

### BRIEF DESCRIPTION OF THE INVENTION

A first object of the present invention refers to a transparent diffusible microemulsion comprising a non-aqueous and an aqueous phase, said non-aqueous phase comprising or consisting of
(a) at least one perfume or essential oil;
(b) at least one nonionic solubilizer;
(c) at least one solvent selected from the group consisting of 1,2-propylenglycol; 3-methoxy-3-methyl-1-butanol (MMB), dipropyleneglycol (DPG), dipropyleneglycol methyl ether (DPM), dipropylene glycol methyl ether acetate (DPMA), and tripropylenglycol methyl ether (TPM);
(d) 1,2-pentanediol.

Surprisingly it has been found, that the compositions show a similar, in some cases improved evaporation performance when compared to non-aqueous diffusible compositions based on dipropyleneglycol monomethyl ether. It was also possible to achieve the same scent performance at lower fragrance/perfume oil concentrations. The compositions are toxicologically save, stable even when stored at elevated temperatures, and work with of sticks made from reed, paper or plastic fibers. Finally, the compositions increase the heart note of the fragrance/perfume oil mixtures, while the scent note caused from DPM, which is not always wanted, is avoided.

In a first preferred embodiment, the transparent microemulsions show a NTU number of at most 1 to 10. The Nephelometric Turbidity Unit (NTU) is a unit used in water treatment for the turbidity of liquids. It is the unit of a liquid's turbidity measured with a calibrated neph-elometer. A NTU number of at most 1 - which is the requirement for drinking water - means a transparent and clear solution with no clouds or hazes. The value is determined at 20 °C, however, the microemulsions according to the present invention show typically a NTU of at most 1 to 10 also when stored at 40 °C and 4°C over a period of 12 weeks.

### Perfume or essential oils

In another preferred embodiment said perfume or essential oils forming component (a) show a high impact. Values are available e.g., threshold on blotter, which is determined by smelling the fragrance raw material in a series of dilutions on a blotter. The concentrated product is diluted with DPG (dipropylene glycol) in a serial order of factors of ten (10% - 1% - 0,1% - 0,01% - 0,001% - 0,0001% - 0,00001% - 0,000001%). Values are given in %. A low value describes a material with a low threshold, a high value one with a high threshold. A panel of 10-12 persons performs the threshold determination by smelling the dilutions on a blotter in comparison to DPG. They start with the maximum dilution/minimum concentration of 0,000001% up to the minimum dilution/maximum concentration of 10%. Scale:
0-9 (as higher, as better)
0 = DPG odorless
1 = 130329 Farnesol 5% in DPG
5 = 130520 Linalool 5% in DPG
9 = 658697 Intreleven aldehyde 5% in DPG

Analysis of the values:
- calculate the average intensity rating of the panelists for each
   solution
- make a graph with the calculated average (concentration in
   logarithmic scale versus intensity)
- use only the (middle) part with the linear slope
- create a line of best fit with gradient and ordinate
- calculate where the graph intersects the intensity of 1 (because 1 is the point, where the raw material starts smelling = threshold)

Oils showing a threshold on blotter of 3 to 9, preferably 5 to 9 and more preferably 7 to 9 allow reducing the amount of perfume material while maintaining the olfactory sensation and creating clear solutions.

Suitable perfume or essential oils are typically mixtures of natural and synthetic perfumes. Natural perfumes include the extracts of blossoms (lily, lavender, rose, jasmine, neroli, ylang-ylang), stems and leaves (geranium, patchouli, petitgrain), fruits (anise, coriander, caraway, juniper), fruit peel (bergamot, lemon, orange), roots (nutmeg, angelica, celery, cardamom, costus, iris, calmus), woods (pinewood, sandalwood, guaiac wood, cedarwood, rosewood), herbs and grasses (tarragon, lemon grass, sage, thyme), needles and branches (spruce, fir, pine, dwarf pine), resins and balsams (galbanum, elemi, benzoin, myrrh, olibanum, opoponax). Animal raw materials, for example civet and beaver, may also be used.

### Nonionic solubilizers

In the following the terms "nonionic solubilizers" and "nonionic emulsifiers" forming component (b) are used synonymously. Suitable nonionic solubilizers encompass:
- products of the addition of 2 to 30 mol ethylene oxide and/or 0 to 5 mol propylene oxide onto linear C₈₋₂₂ fatty alcohols, onto C₁₂₋₂₂ fatty acids and onto alkyl phenols containing 8 to 15 carbon atoms in the alkyl group;
- C_{12/18} fatty acid monoesters and diesters of addition products of 1 to 30 mol ethylene oxide onto glycerol;
- glycerol mono- and diesters and sorbitan mono- and diesters of saturated and unsaturated fatty acids containing 6 to 22 carbon atoms and ethylene oxide addition products thereof;
- addition products of 15 to 60 mol ethylene oxide onto castor oil and/or hydrogenated castor oil;
- polyol esters and, in particular, polyglycerol esters such as, for example, polyglycerol polyricinoleate, polyglycerol poly-12-hydroxystearate or polyglycerol dimerate isostearate. Mixtures of compounds from several of these classes are also suitable;
- addition products of 2 to 15 mol ethylene oxide onto castor oil and/or hydrogenated castor oil;
- partial esters based on linear, branched, unsaturated or saturated C_{6/22} fatty acids, ricinoleic acid and 12-hydroxystearic acid and glycerol, polyglycerol, pentaerythritol, - dipentaerythritol, sugar alcohols (for example sorbitol), alkyl glucosides (for example methyl glucoside, butyl glucoside, lauryl glucoside) and polyglucosides (for example cellulose);
- mono-, di and trialkyl phosphates and mono-, di- and/or tri-PEG-alkyl phosphates and salts thereof;
- wool wax alcohols;
- polysiloxane/polyalkyl polyether copolymers and corresponding derivatives;
- mixed esters of pentaerythritol, fatty acids, citric acid and fatty alcohol and/or mixed esters of C₆₋₂₂ fatty acids, methyl glucose and polyols, preferably glycerol or polyglycerol,
- alkyl and/or alkenyl oligoglycosides;
- polyalkylene glycols and
- glycerol carbonate.

The addition products of ethylene oxide and/or propylene oxide onto fatty alcohols, fatty acids, alkylphenols, glycerol mono- and diesters and sorbitan mono- and diesters of fatty acids or onto castor oil are known commercially available products. They are homologue mixtures of which the average degree of alkoxylation corresponds to the ratio between the quantities of ethylene oxide and/or propylene oxide and substrate with which the addition reaction is carried out. C_{12/18} fatty acid monoesters and diesters of addition products of ethylene oxide onto glycerol are known as lipid layer enhancers for cosmetic formulations. The preferred emulsifiers are described in more detail as follows:

**Alcohol alkoxylates.** Suitable alcohol alkoxylates, also called alkyl polyglycol ethers follow for example formula (I)

R¹O(EO)ₓ(PO)_{y}H (I)

wherein R¹ stands for a linear or branched saturated or unsaturated alkyl or alkenyl group with 6 to 16 carbon atoms, EO for an ethylene oxide unit, PO for a propylene oxide unit, x and y independently from each other for 0 or an integer from 1 to 20, and the sum (x+y) is different from 0. Particularly preferred are adducts of 1 to 10, preferably 5 to 9 ethylene oxide units to fatty alcohols with linear structure such as lauryl alcohol, myristyl alcohol, cetyl alcohol or stearyl alcohol or oxo-alcohols such as tridecanol. Particularly preferred are the products Trideceth-7 and Trideceth-9.

**Sorbitan esters.** Suitable sorbitan esters are sorbitan monoisostearate, sorbitan sesquiisostearate, sorbitan diisostearate, sorbitan triisostearate, sorbitan monooleate, sorbitan sesquioleate, sorbitan dioleate, sorbitan trioleate, sorbitan monoerucate, sorbitan sesquierucate, sorbitan dierucate, sorbitan trierucate, sorbitan monoricinoleate, sorbitan sesquiricinoleate, sorbitan diricinoleate, sorbitan triricinoleate, sorbitan monohydroxystearate, sorbitan ses-quihydroxystearate, sorbitan dihydroxystearate, sorbitan trihydroxystearate, sorbitan monotartrate, sorbitan sesquitartrate, sorbitan ditartrate, sorbitan tritartrate, sorbitan monocitrate, sorbitan sesquicitrate, sorbitan dicitrate, sorbitan tricitrate, sorbitan monomaleate, sorbitan sesquimaleate, sorbitan dimaleate, sorbitan trimaleate and technical mixtures thereof. Addition products of 1 to 100 and preferably 20 to 80 mol ethylene oxide onto the sorbitan esters mentioned are also suitable. Alkoxylated sorbitan esters are also called polysorbates and are sold for example under the tradename "Tween^{®}20, 40 or 60". For example Tween 20 stands for a sorbitanmonolaurate+20EO; the product is also called polysorbate-20.

**Polyglycerol esters.** Typical examples of suitable polyglycerol esters are Polyglyceryl-2 Dipolyhydroxystearate (Dehymuls^{®} PGPH), Polyglycerin-3-Diisostearate (Lameform^{®} TGI), Polyglyceryl-4 Isostearate (Isolan^{®} Gl 34), Polyglyceryl-3 Oleate, Diisostearoyl Polyglyceryl-3 Diisostearate (Isolan^{®} PDI), Polyglyceryl-3 Methylglucose Distearate (Tego Care^{®} 450), Polyglyceryl-3 Beeswax (Cera Bellina^{®}), Polyglyceryl-4 Caprate (Polyglycerol Caprate T2010/90), Polyglyceryl-3 Cetyl Ether (Chimexane^{®} NL), Polyglyceryl-3 Distearate (Cremophor^{®} GS 32) and Polyglyceryl Polyricinoleate (Admul^{®} WOL 1403), Polyglyceryl Dimerate Isostearate and mixtures thereof. Examples of other suitable polyolesters are the mono-, di- and triesters of trimethylol propane or pentaerythritol with lauric acid, cocofatty acid, tallow fatty acid, palmitic acid, stearic acid, oleic acid, behenic acid and the like optionally reacted with 1 to 30 mol ethylene oxide.

**Alkyl and/or alkenyl oligoglycosides.** Alkyl and/or alkenyl oligoglycosides, their preparation and their use are known from the prior art. They are prepared in particular by reacting glucose or oligosaccharides with primary alcohols containing 8 to 18 carbon atoms. With regard to the glycoside residue, monoglycosides, in which a cyclic sugar residue is glyco-sidically bonded to the fatty alcohol, as well as oligomeric glycosides with a degree of oligomerization up to preferably about 8 are suitable. The degree of oligomerization is a statistical mean value based on a homolog distribution customary for such technical products.

### Alkoxylated castor oils

This group of suitable solubilizers includes hardened and unhardened castor oil. The degree of alkoxylation is typically between 2 and 100, with ethylene oxide addition products being used almost exclusively. PEG40 hydrogenated castor oil is particularly preferred and is available, for example, under the tradename Cremophor^{®} CO40 (BASF).

### Solvents

According to the present invention, two different solvents need to be present. Component (c) is thereby selected from the group consisting of 1,2-propylenglycol; 3-methoxy-3-methyl-1-butanol (MMB), dipropyleneglycol (DPG), dipropyleneglycol methyl ether (DPM), and tripropylenglycol methyl ether (TPM).

Component (d) consists of pentylene glycol (synonym: 1,2-pentanediol). With regard to pentylene glycol the product Hydrolite 5 Green (Symrise AG) is particularly preferred, since it is manufactured based on renewable sources.

The selection of the right solvent for the microemulsion, is a very important part for later evaporation behaviour. The solvent plays an important role, so that less emulsifier can be used. For the water-based reed, a mixture of Hydrolite-green^{®} (3%) and propylene glycol (2,5%-6%) has been found advantageous. The combination of these solvents, mixed with the emulsifier, showed the best solubility. These solvents are also beneficial, because they evaporate slowly but constantly, this optimises the evaporation rate of the system.

### Microemulsions

A microemulsion is an emulsion whose disperse phase (e.g., oil or water) forms such small domains ("droplets") that visible light is not scattered by them. This causes microemulsions to be transparent like water, while normal emulsions are opaque like milk. The domain diameter is in the lower nanometer range (< 350 nm). A typical microemulsion consists of two immiscible liquid components and a surface-active solubilizer. At sufficiently high concentrations, the latter forms micelles in an aqueous environment, in which the surface-active molecules are arranged in such a way that the lipophilic part is always oriented inward and the hydrophilic molecule part is always oriented outward. This allows the micelles to hold the lipophilic component inside them, while the hydrophilic molecule part interacts more strongly with the hydrophilic component.

Keeping this in mind, the microemulsion according to the present invention typically comprise:
(a) about 2 to about 5 wt.-percent of at least one fragrance or perfume oil;
(b) about 3 to about 7 wt.-percent of at least one nonionic solubilizer;
(c) about 2 to about 7 wt.-percent 1of at least one solvent;
(d) about 2 to about 4 wt.-percent 1,2-pentanediol;
on condition that all amounts add with water to 100 wt.-percent.

More preferably the microemulsion comprise:
(a) about 3 to about 5 wt.-percent of at least one fragrance or perfume oil;
(b) about 3.5 to about 5.0 wt.-percent of at least one nonionic solubilizer;
(c) about 2.5 to about 6.0 wt.-percent of at least one solvent;
(d) about 2.5 to about 3.5 wt.-percent 1,2-pentanediol;
on condition that all amounts add with water to 100 wt.-percent.

Particularly preferred are microemulsions, wherein the perfume oil has a fruity, green, woody, amber, or musk scent note and the nonionic solubilizer is selected from the group consisting of trideceth-9, polysorbate-20, PEG40 hydrogenated castor oil or mixtures thereof.

Also claimed is a process for obtaining a transparent diffusible microemulsion comprising or consisting of the following steps:
(i) providing a non-aqueous phase comprising or consisting of at least one perfume oil, at least one nonionic emulsifier, at least one solvent and 1,2-pentanediol;
(ii) providing an aqueous phase;
(iii) blending said non-aqueous and said aqueous phase and subjecting the mixture to stirring unless a transparent emulsion showing a NTU of at most 10 is formed.

Preferably, the aqueous phase is added to the mixture part by part.

### INDUSTRIAL APPLICATION

Another object of the present invention refers to A method for air scenting comprising or consisting of the following steps:
(i) providing a microemulsion as described above;
(ii) diffusing said microemulsion by means of sticks made from reed, paper or plastic fiber.

Also claimed is use of the microemulsion as described above as an aroma or fragrance diffuser composition.

A final embodiment of the present invention refers to a scent kit - also referred to as a "reed diffusor" - consisting of a diffusor, the microemulsion as described above and at least one stick made from reed, paper or plastic fiber.

For the sake of good order and to avoid ambiguities it is expressly pointed out that the same preferred embodiments, substances, mixtures, ratios and so on apply to the claimed embodiments for industrial applicability as have already been described above. A repetition of these enumerations is therefore superfluous.

### EXAMPLES

### Example 1

### Evaporation experiments

6 microemulsions were prepared using the same aqueous system of solubilizers and solvents, but different perfume oils. The compositions are provided in **Table 1:**

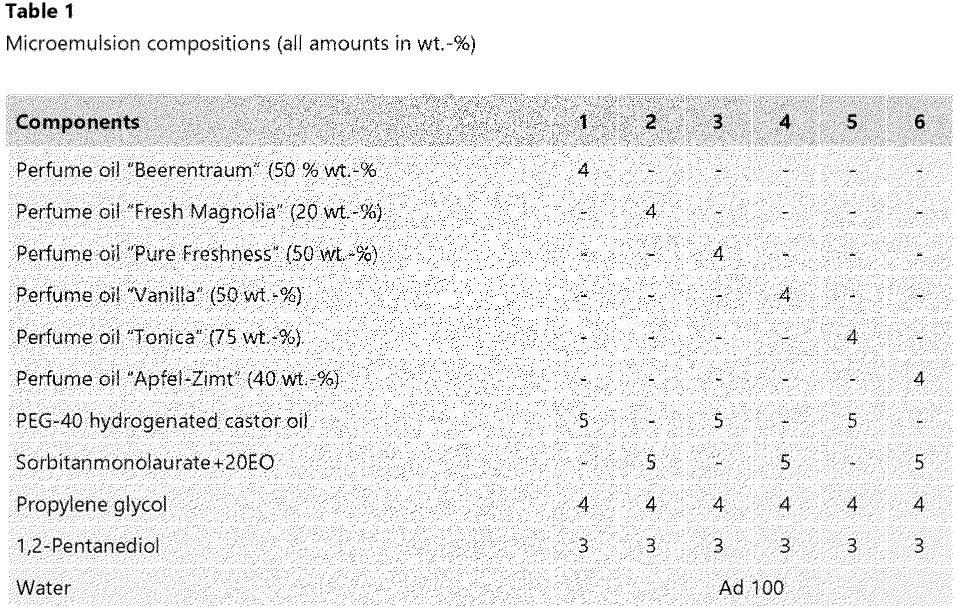

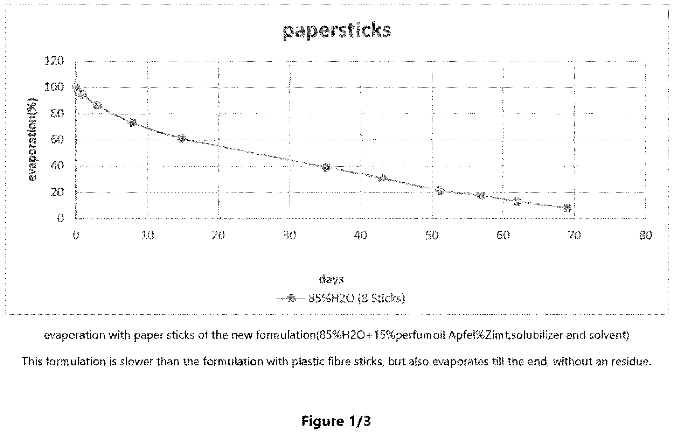

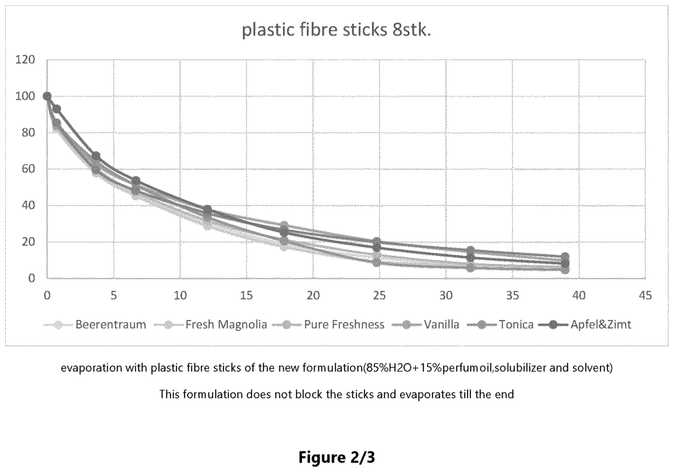

The evaporation tests were conducted with 90 g samples of each microemulsion and 5 paper sticks of 20 cm. The test was carried out over a period of 40 to 80 days at 20 °C. Determined has been the remaining amount of microemulsion in the diffusor. The results are depicted in **Figures 1** **and** **2****.**

### Comparative Example 1

### Evaporation experiments

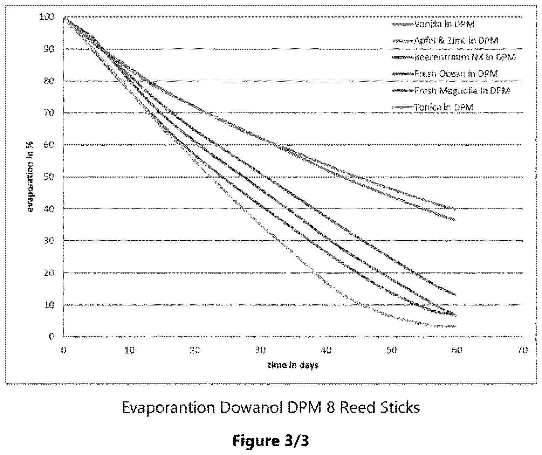

For comparison inventive example 1 has been repeated however using 6 reed diffusor compositions based on dipropropyleneglycol monomethylether (Dowanol DPM) as the solvent, and using both paper and plastic sticks. The results are depicted in **Figure 3****.**

## Claims

1. A transparent diffusible microemulsion comprising a non-aqueous and an aqueous phase, said non-aqueous phase comprising or consisting of
(a) at least one fragrance or perfume oil;
(b) at least one nonionic solubilizer;
(c) at least one solvent selected from the group consisting of 1,2-propylenglycol; 3-methoxy-3-methyl-1-butanol (MMB), dipropyleneglycol (DPG), dipropyleneglycol methyl ether (DPM), dipropylene glycol methyl ether acetate (DPMA), and tripropylenglycol methyl ether (TPM); and
(d) 1,2-pentanediol.

2. The microemulsion of Claim 1 showing a nephelometric turbidity (NTU) of at most 1 to 10.

3. The microemulsion of Claim 1, wherein said perfume oils show threshold on blotter values of from about 3 to about 9.

4. The microemulsion of Claim 1, wherein said perfume oils represent extracts of plant and animal sources selected from the group consisting of lily, lavender, rose, jasmine, neroli, ylang-ylang, geranium, patchouli, petitgrain, anise, coriander, caraway, juniper, bergamot, lemon, orange, nutmeg, angelica, celery, cardamom, costus, iris, calmus, pinewood, sandalwood, guaiac wood, cedarwood, rosewood, tarragon, lemon grass, sage, thyme, spruce, fir, pine, dwarf pine, galbanum, elemi, benzoin, myrrh, olibanum, opoponax, civet, beaver and mixtures thereof.

5. The microemulsion of Claim 1, wherein said nonionic solubilizers are selected from the group consisting of alkoxylated alcohols, sorbitan esters, polyglycerol esters, alk(en)yl oligoglycosides and alkoxylated castor oils.

6. The microemulsion of Claim 6, wherein the alkoxylated alcohols follow formula (I)
R¹O(EO)ₓ(PO)_{y}H (I)
wherein R1 stands for a linear or branched saturated or unsaturated alkyl or alkenyl group with 6 to 16 carbon atoms, EO for an ethylene oxide unit, PO for a propylene oxide unit, x and y independently from each other for 0 or an integer from 1 to 20, and the sum (x+y) is different from 0.

7. The microemulsion of Claim 6, wherein the sorbitan esters are selected from the group consisting of sorbitan monoisostearate, sorbitan sesquiisostearate, sorbitan diisostearate, sorbitan triisostearate, sorbitan monooleate, sorbitan sesquioleate, sorbitan dioleate, sorbitan trioleate, sorbitan monoerucate, sorbitan sesquierucate, sorbitan dierucate, sorbitan trierucate, sorbitan monoricinoleate, sorbitan sesquiricinoleate, sorbitan diricinoleate, sorbitan triricinoleate, sorbitan monohydroxystearate, sorbitan ses-quihydroxystearate, sorbitan dihydroxystearate, sorbitan trihydroxystearate, sorbitan monotartrate, sorbitan sesquitartrate, sorbitan ditartrate, sorbitan tritartrate, sorbitan monocitrate, sorbitan sesquicitrate, sorbitan dicitrate, sorbitan tricitrate, sorbitan monomaleate, sorbitan sesquimaleate, sorbitan dimaleate, sorbitan trimaleate including its addition products of 10 to 100 mol ethylene oxide.

8. The microemulsion of Claim 6, wherein the alkoxylation product of castor oil is PEG40 hydrogenated castor oil.

9. The microemulsion of Claim 1, wherein the perfume oil has a fruity, green, woody, amber, or musk scent note and the nonionic solubilizer is selected from the group consisting of trideceth-9, polysorbate-20, PEG40 hydrogenated castor oil or mixtures thereof.

10. The microemulsion of Claim 1, comprising
(a) about 3 to about 5 wt.-percent of at least one fragrance or perfume oil;
(b) about 3 to about 7 wt.-percent of at least one nonionic solubilizer;
(c) about 2 to about 7 wt.-percent of at least one solvent;
(d) about 2 to about 4 wt.-percent 1,2-pentanediol;
on condition that all amounts add with water to 100 wt.-percent.

11. The microemulsion of Claim 1, comprising
(a) about 3 to 5 wt.-percent of at least one fragrance or perfume oil;
(b) about 3.5 to about 5.0 wt.-percent of at least one nonionic solubilizer;
(c) about 2.5 to about 6.0 wt.-percent of at least one solvent; and
(d) about 2.5 to about 3.5 wt.-percent 1,2-pentanediol;
on condition that all amounts add with water to 100 wt.-percent.

12. A process for obtaining a transparent diffusible microemulsion comprising or consisting of the following steps:
(i) providing a non-aqueous phase comprising or consisting of at least one perfume oil, at least one nonionic emulsifier, at least one solvent and 1,2-pentanediol;
(ii) providing an aqueous phase;
(iii) blending said non-aqueous and said aqueous phase and subjecting the mixture to stirring unless a transparent emulsion showing a NTU of at most 1 to 10 is formed.

13. A method for air scenting comprising or consisting of the following steps:
(i) providing a microemulsion according to Claim 1;
(ii) diffusing said microemulsion by means of sticks made from reed, paper or plastic.

14. The use of the microemulsion of Claim 1 as an aroma or fragrance diffuser composition.

15. A scent kit consisting of a diffusor, the microemulsion of Claim 1 and at least one stick made from reed, paper or plastic:
